# EUROPÄISCHE PATENTSCHRIFT

(11) **EP 1 194 113 B1**
(45) Veröffentlichungstag und Bekanntmachung des Hinweises auf die Patenterteilung: **09.03.2011**
(21) Anmeldenummer: 00956075.6
(22) Anmeldetag: 07.07.2000
(51) Int. Cl.: A61K 8/19, A61K 8/21, A61K 8/24, A61K 8/25, A61Q 1/02, A61Q 19/00, C03C 4/00, C03C 3/097, C03C 3/11

(54) **Verwendung von bioaktivem Glas zur Konservierung von kosmetischen und pharmazeutischen Präparaten**
Use of bioactive glass as a preservative for cosmetic and pharmaceutical preparations
Utilisation d'un verre bioactif en tant qu'agent conservateur pour préparations cosmétiques et pharmaceutiques

(30) Priorität: 09.07.1999 DE 19932239
(43) Veröffentlichungstag der Anmeldung: 10.04.2002
(73) Patentinhaber: Schott AG, 55122 Mainz (DE)
(72) Erfinder: Kessler, Susanne, D-84030 Ergolding (DE); LEE, Sean, 76227 Karlsruhe (DE)
(74) Vertreter: Fritzsche, Thomas
(86) Internationale Anmeldenummer: PCT/DE2000/002231
(87) Internationale Veröffentlichungsnummer: WO 2001/003650

(56) Entgegenhaltungen:
- WO-A-96/24364
- WO-A1-00/76486
- WO-A1-97/27148
- US-A- 4 155 870
- US-A- 5 074 916
- US-A- 5 766 611
- PATENT ABSTRACTS OF JAPAN vol. 0152, no. 78, 15. Juli 1991 (1991-07-15) & JP 03 095516 A (ADVANCE), 19. April 1991 (1991-04-19)
- PATENT ABSTRACTS OF JAPAN vol. 1998, no. 10, 31. August 1998 (1998-08-31) & JP 10 139644 A (SUNSTAR), 26. Mai 1998 (1998-05-26)
- HENCH L.L.: 'Bioceramics: From Concept to Clinic' J. AM. CERAM. SOC. Bd. 74, Nr. 7, Juli 1991, MALDEN, MA, US, Seiten 1487 - 1510, XP002090879

## Beschreibung

Verwendung von bioaktivem Glas zur Konservierung von kosmetischen und pharmazeutischen Präperaten

### Beschreibung:

Die Erfindung betrifft die Verwendung von bioaktivem Glas zur Konservierung von kosmetischen und pharmazeutischen präperaten.

Es ist bekannt, eine Vielzahl von verderblichen Waren, wie Lebensmittel, Arzneimittel etc. mittels chemischen, insbesondere organo-chemischen Konservierungsmitteln zu behandeln, um ihre Verwendungsdauer zu verlängern.

Dies gilt insbesondere auch für kosmetische Präparate, die mit der Haut in Kontakt kommen. Auf diese Weise werden nämlich beim Auftragen auf die Haut oder insbesondere bei Dosen durch Entnahme mit der Hand, oder auch beim Gebrauch von Lippenstiften Bakterien in die Präparate eingetragen. Durch das in kosmetischen Zubereitungen herrschende hautverträgliche Milieu können sich die so eingebrachten Bakterien rasch vermehren, so daß schon nach wenigen Tagen ihre Konzentration unverträglich hoch sein kann. Das gleiche Problem tritt auch bei kosmetischen Präparaten auf, die in Flaschen oder Tuben aufbewahrt werden. Zwar ist hier durch den geringeren Kontakt mit der Haut bei der Entnahme die Kontamination mit Bakterien geringer, jedoch läßt sich auch hier ein Kontakt der Bakterien tragenden Haut mit dem Präparat nicht vermeiden, so daß es auch bei diesen Darreichungsformen zu einer raschen Kontaminierung kommt. Aus diesem Grunde ist es notwendig, derartige handelsübliche Präparate mit Bakteriziden bzw. bakteriostatischen Mitteln zu versehen. Derartige Mittel sind jedoch zytotoxisch und wirken darüber hinaus bei einer großen Anzahl von Menschen allergen.

Um hierzu weltweit eine sachgerechte Herstellungspraxis sicherzustellen, hat die WHO bereits 1968 die sogenannte "Good Manufacturing Practice" (GMP) erlassen. Nach dieser Richtlinie soll unter anderem Produkthygiene, sowie Qualitätskontrolle sichergestellt-und Verunreinigungen vermieden werden. Inzwischen ist die GMP noch durch die, insbesondere in der pharmazeutischen und kosmetischen Industrie gültige "Good Storage Practice" (GSP) ergänzt worden, welche eine produktgerechte Lagerung sicherstellen soll.

In der US-A 5,766,611 ist daher bereits versucht worden, ein lösliches Glas als Konservierungsmittel zu verwenden, das Silber, Zink und/oder Kupfer freisetzt. Jedoch wirken auch diese aus dem Glas freigesetzten Substanzen als Zellgift und sind zytotoxisch. Der ständige Kontakt mit einem derartigen Konservierungsmittel ist somit nicht unbedenklich.

Es ist bereits bekannt, sog. bioaktives Glas zur Mundpflege zu verwenden. So sind beispielsweise Zahnpasten bekannt, welche die Remineralisierung der Zähne bewirken. In der US-A-5, 834, 008 ist die Verwendung von Bioglas zur Wundheilung beschrieben. So lassen sich beispielsweise nicht heilende, ulcerierende Wunden, insbesondere bei Diabetikern, und Schlaganfallpatienten durch die Applikation derartiger Glassorten zum Abheilen bringen.

J. Allen, H. Neumann und M. Wilson beschreiben die Inhibierung bakterieller Ansiedelungen durch Bioglas^{®} 45S5 mit einer Korngröße von 355 - 500µm, wobei normale Glasbeads (Fensterglas) mit einer vergleichbaren Korngröße von 455 - 600 µm keinerlei antibakterielle Wirkung zeigt.

Die wesentlichen Eigenschaften von bioaktivem Glas sind dem Fachmann bekannt und beispielsweise in der US-A 5,074,916 beschrieben. Danach unterscheidet sich bioaktives Glas von herkömmlichen Kalk-Natrium-Silikat - Gläsern dadurch, dass es lebendes-Gewebe bindet.

Bioaktives Glas ist beispielsweise unter der Bezeichnung "Bioglas^{®}" von USBiomaterials Corp. Alachua, Fl 32615, USA erhältlich und bezeichnet ein Glas, das eine spezielle biologische Reaktion bei Kontakt mit einem Organismus zeigt. Beim Zusammentreffen mit Körperflüssigkeit tauscht es mit dieser Ionen aus. Bei dem Ionenaustausch werden Natrium- und Calciumionen freigesetzt, die auf dem Glas innerhalb weniger Minuten eine Kieselgelschicht ausbilden, die die Ausbildung von einer Hydroxy-Carbonat-Apatitschicht bewirkt und die mit der mineralischen Phase des Knochens identisch ist. Darauf wird das Hydroxy-Carbonat-Apatit gebildet bzw. Zellen produzieren Kollagen, welches in diese Schicht eingebettet wird. Dies führt zu einer physio-chemischen Bindung zwischen dem bioaktiven Glas, dem Gewebe oder dem Knochenmaterial, was eine schnellere Regenerierung des Knochens bewirkt. Dabei konnte in Tierstudien gezeigt werden, daß Bioglas^{®} annähernd gleich wirksam ist wie eine Transplantation mit eigenen Knochenteilen.

Auch die Verwendung von bioaktivem Glas zur Aknebekämpfung ist bekannt. Dabei wird ein ein bioaktives Glas enthaltendes Präparat auf die mit Akne befallenen Hautstellen aufgetragen, wodurch die Entzündungen positiv beeinflußt werden. In diesem Zusammenhang ist auch die Publikation von John E. Rectenwald, L. L. Moldawer, Sean Lee et al. in Infection and Immunity (zur Publikation eingereicht) sowie 19th Annual Meeting, Surgical Infection Society, April 29 bis May 1 1999 Seattle zu erwähnen, worin beschrieben ist, daß Bioglas die Bildung bzw. Ausschüttung des entzündungshemmenden Hormons Interleukin 6 (IL 6) bewirkt und die entzündungssteigernden Cytokine TNF-α, IL-1-α und IL-10 unterdrückt.

Darüber hinaus ist von E. Allen, et. al. (Departments of Microbiology in Periodontology Eastman Dental Institut) bekannt, daß ein bioaktives Glas 45-S-5 welches von Bioglas^{®} U.S. Biomaterials Alachua, FL. 32615 USA erhältlich ist, eine antibakterielle Wirkung zeigt, welche jedoch nicht mit normalen Glaskügelchen, sog. Glasbeads (Fensterglas) erreicht werden kann.

Die Erfindung hat nun zum Ziel ein Konservierungsmittel bereitzustellen, welches im Gegensatz zu den bislang bekannten Mitteln keine negativen Wirkungen bei der Applikation, insbesondere bei der Applikation auf der Haut zeigt.

Dieses Ziel wird nun erfindungsgemäß durch die im Hauptanspruch definierten Maßnahmen

Bioaktive Gläser sind bereits seit langem bekannt und beispielsweise zusammenfassend von Larry L. Hench und John K. West in "Biological Applications of Bioactive Glasses", Life Chemistry Reports 1996, vol. 13, p. 187 - 241 oder in "An Introduction to Bioceramics", L. Hench und J. Wilson, eds. World Scientific, New Jersey (1993) beschrieben. Bioaktive Gläser zeichnen sich im Gegensatz zu herkömmlichen Gläsern dadurch aus, dass diese in einem wässrigen Medium löslich sind und an ihrer Oberfläche eine Hydroxylapatitschicht ausbilden. Die gängigsten bioaktiven Gläser werden entweder als Schmelzglas hergestellt, wobei diese dann gegenüber normalen Fenster- oder Flaschengläsern einen deutlich geringeren Anteil an SiO₂ und einen wesentlich höheren Anteil an Natrium aufweisen oder sie sind sogenannte Sol-Gel-Gläser, welche dann, im Gegensatz zu Schmelzgläsern einen hohen Anteil von Siliziumoxid sowie einen geringen bis gar keinen Anteil an Natrium enthalten können.

Bioaktives Silizium enthaltendes Glas bzw. bioaktives Glas bezieht sich auf ein Material, welches Siliziumoxid oder Siliziumhydroxyd umfaßt, wobei dieses Material die Bildung und Übertragung von SiOH-Gruppen ermöglicht. Bioaktives Silizium enthaltendes Glas kann daher beispielsweise ein bioaktives Glas aus einer Mischung von Siliziumoxid oder Siliziumhydroxid mit einem oder mehreren Elementen der Gruppe umfassend Natrium, Kalium, Calcium, Magnesium, Bor, Titan, Aluminium sowie Stickstoff, Phosphor und Fluor enthaltenden Anionen sein. Darüber hinaus kann es beispielsweise Natriumsilikate des Wasserglastypes oder Kieselgele sowie SiOH-Gruppen enthaltende Lösungen oder auch Hydroxyapatit umfassenden Siliziumoxide oder Siliziumhydroxide sowie Kieselgel, welches Calcium und Phosphor enthält, sein. Eines der wesentlich gemeinsamen Merkmale von bioaktivem Kieselgel enthaltenden Glas zeigt sich durch die Bildung und Übertragung von SiOH-Gruppen. Darüber hinaus sollten auch Calcium und Phosphationen enthalten sein.

Die erfindungsgemäß verwendeten bioaktiven Gläser sind vorzugsweise ein herkömmliches bioaktives Glas, welches dem Fachmann bestens bekannt ist. Solche Gläser enthalten üblicherweise SiO₂, einen hohen Anteil an Na₂O und CaO sowie Phosphor und zwar in einem hohen Molverhältnis von calcium zu Phosphor, welches sich meist, jedoch nicht notwendigerweise um etwa 5 bewegt. Kommen solche bioaktiven Gläser mit Wasser oder einer Körperflüssigkeit in Kontakt, dann zeichnen sie sich durch spezielle Reaktionen aus, und zwar werden dabei Natrium- und Calciumionen des Glases durch H⁺-Ionen aus der Lösung in Form einer Kationen-Austauschreaktion ersetzt, wodurch eine Silanol-Gruppen aufweisende Oberfläche entsteht, an welche sich Natrium- und Calciumhydroxid anlagern. Die Erhöhung der Hydroxy-Ionenkonzentration führt an der Glasoberfläche nun zu einer weiteren Reaktion mit dem SiO₂-Netzwerk, wodurch weitere Silanolgruppen entstehen, die auch tiefer im Glas liegen können.

Aufgrund des hohen alkalischen pH im Glaszwischenraum entsteht aus CaO und P₂O₅ eine gemischte Hydroxylapatit-Phase, welche auf der SiO₂-Oberfläche auskristallisiert und in biologischen Materialien mit Mucopolysacchariden, Kollagenen und Glycoproteinen bindet.

Das Molverhältnis von Calcium zu Phosphor ist vorzugsweise > 2 und insbesondere > 3 und ist vorzugsweise < 30, insbesondere < 20, wobei Verhältnisse von < 10 besonders bevorzugt sind.

Bei einem bioaktiven Glas, welches ein Schmelzglas ist, dann liegt die Obergrenze an enthaltendem Siliziumdioxid bei 60 vorzugsweise bei 55 Gew.%, wobei eine Obergrenze von 50 Gew.% besonders bevorzugt ist. Der Gehalt an Natriumoxid beträgt vorzugsweise mehr als 15 Gew.%, insbesondere mehr als 18 Gew.%. Ein Natriumoxid-Gehalt von > 20 Gew.% ist besonders bevorzugt.

Der Gehalt an Phosphoroxid beträgt bei bioaktiven Gläsern mindestens 2 Gew.%, insbesondere mindestens 4 Gew.%.

In einer bevorzugten Ausführungsform weist das lösliche bioaktive Glas keine toxisch wirkenden Metallkationen wie Ag⁺, Cu²⁺, Cu⁺ und/oder Zn²⁺ etc. auf bzw. setzt solche nicht in toxisch wirkenden Konzentrationen frei. Zur Erreichung von synergistischen Effekten kann es jedoch in Einzelfällen erwünscht sein, auch solche biozid wirkenden Gläser zuzusetzen, welche toxisch wirkende Kationen freisetzen.

Bei bioaktivem Glas selbst handelt es sich um ein Material, welches eine mehr oder weniger runde Form, wie beispielsweise Sand, aufweist. Derartige Teilchen können eine Größe bis zu ca. 0,5-1 mm aufweisen, sind jedoch vorzugsweise wesentlich kleiner. Übliche Teilchengrößen sind ≤ 400 µm und insbesondere ≤ 200µm, als besonders zweckmäßig haben sich Teilchengrößen ≤ 100 µm, vorzugsweise ≤ 90 µm, und im besonderen ≤ 60µm bzw. ≤ 20µm erwiesen. Eine bevorzugte Körnung-weist einen Durchmesser d₅₀ von ≤ 10 µm, vorzugsweise ≤ 5 µm, insbesondere ≤ 2 µm auf. Je höher das Verhältnis von Oberfläche zu Gewicht bzw. Volumen ist, um so höher ist auch die biozide Wirkung der Partikel.

In einer bevorzugten erfindungsgemäßen Ausführungsform wird bioaktives Glas zur Konservierung von kosmetischen Präparaten verwendet. Bevorzugt wird hierbei feinkörniges bioaktives Glas mit einer Körnung von d₅₀ ≤ 5 µm, insbesondere ≤ 2 µm verwendet. Bevorzugte kosmetische Präparate sind insbesondere Cremes, Make-up-Mittel, Lippenstifte sowie Lotions und Salben. Durch die in den Präparaten enthaltene Feuchtigkeit wird die mikrobiozide Wirkung von bioaktivem Glas weiter gefördert.

Das erfindungsgemäß verwendete biozide Konservierungsmittel ist vorzugsweise in einer Menge von bis zu 25 Gew.%, insbesondere bis 10 Gew.% bezogen auf den Feststoffgehalt des zu konservierenden Präparates enthalten. Bevorzugt sind jedoch Obergrenzen von 7 Gew.% bzw. 5 Gew.%, wobei 3 Gew.% besonders bevorzugt sind. Untere Grenzwerte sind 0,01 Gew.%, insbesondere 0,1 Gew.%, wobei 0,5 Gew.% bzw. 1 Gew.% als untere wirksame Menge besonders bevorzugt sind.

Es hat sich überraschenderweise gezeigt, daß sich mit dem bioaktiven Glas derartige Präparate hervorragend konservieren lassen, ohne daß den Mitteln hautirritierende zytotoxische und ggf. allergen wirkende chemische Konservierungsmittel zugesetzt werden müssen. Darüber hinaus wird ein zusätzlicher Pflegeeffekt durch die heilungsfördernde, insbesondere auch entzündungshemmende Wirkung von bioaktivem Glas erzielt.

In besonderen Fällen kann es jedoch auch erwünscht sein, das erfindungsgemäße Konservierungsmittel zu Präparationen zuzusetzen, die mittels üblichen Konservierungsmitteln haltbargemacht sind, um synergistische Effekte zu erzielen.

Das Konservierungsmittel wird vorzugsweise in protonenhaltigen Lösungsmitteln insbesondere in wässrigen und/oder in alkoholischen Lösungsmitteln eingesetzt. Es ist jedoch auch möglich, daß Konservierungsmittel in aprotonischen Lösungsmittel zu verwenden, sofern das dortige Milieu die Freisetzung der löslichen Kationen des Glases ermöglicht. Bevorzugte aprotische Lösungsmittel sind Ketone wie Aceton, Fette, Wachse, Öle sowie entsprechende flüssige oder feste Kohlenwasserstoffe. Besonders geeignet ist das erfindungsgemäße Konservierungsmittel auch für die Konservierung von Wasser-in-öl- sowie Öl-in-Wasser-Emulsionen.

Auch flüssige kosmetische Präparate, deren Alkoholanteil zur Konservierung nicht ausreicht, lassen sich mittels bioaktivem Glas konservieren. Hierbei ist es bevorzugt, ein Glas zu verwenden, dessen Brechungsindex demjenigen der Flüssigkeit entspricht. Auf diese Weise wird das zugesetzte Glas für den Anwender "unsichtbar". Es ist jedoch auch möglich, das Glas bewußt in einer gewünschten Weise sichtbar zu machen, um kosmetische Effekte zu erreichen, beispielsweise durch Einfärbung.

## Patentansprüche

1. Verwendung von bioaktivem Glas, das bezogen auf das Gesamtglasgewicht 40 - 60 Gew.-% SiO₂, 10 - 30 Gew. -% CaO, 10 - 35 Gew.-% Na₂O, 2 - 8 Gew.-% P₂O₅, 0 - 25 Gew. -% CaF₂, 0 - 10 Gew. -% B₂O₃, 0 - 8 Gew. -% K₂O, und 0 - 5 Gew.-% MgO umfasst. zur Konservierung von kosmetischen und/oder pharmazeutischen Präparaten.

2. Verwendung nach Anspruch 1, **dadurch gekennzeichnet, dass** das bioaktive Glas zusammen mit einem protischen Lösungsmittel verwendet wird.

3. Verwendung nach Anspruch 1 oder 2, **dadurch gekennzeichnet, dass** das Präparat ein Creme, eine Lotion, ein Lippenstift, eine Make-up-Zusammensetzung und/oder eine Tinktur ist.

## Claims

1. Use of bioactive glass, which comprises, based on the total weight of glass, 40-60% by weight of SiO₂, 10-30% by weight of CaO, 10-35% by weight of Na₂O, 2-8% by weight of P₂O₅, 0-25% by weight of CaF₂, 0-10% by weight of B₂O₃, 0-8% by weight of K₂O and 0-5% by weight of MgO, for the preservation of cosmetic and/or pharmaceutical preparations.

2. Use according to Claim 1, **characterized in that** the bioactive glass is used together with a protic solvent.

3. Use according to Claim 1 or 2, **characterized in that** the preparation is a cream, a lotion, a lipstick, a make-up composition and/or a tincture.

## Revendications

1. Utilisation de verre bio actif, qui comprend, par rapport au poids de verre total, 40 -60 % en poids de SiO₂, 10 - 30 % en poids de CaO, 10 - 35% en poids de Na₂O, 2 - 8 % en poids de P₂O₅, 0 - 25 % en poids de CaF₂, 0 - 10 % en poids de B₂O₃, 0 - 8 % en poids de K₂O et 0 - 5 % en poids de MgO, en vue de la conservation de préparations cosmétiques et/ou pharmaceutiques.

2. Utilisation selon la revendication 1, **caractérisée en ce que** le verre bio actif est utilisé conjointement à un solvant protique.

3. Utilisation selon la revendication 1 ou 2, **caractérisée en ce que** la préparation est une crème, une lotion, un rouge à lèvres, une composition de maquillage et/ou une teinture.
